# EUROPEAN PATENT APPLICATION

(11) **EP 4 813 333 A1**
(43) Date of publication of application: **30.09.2026**
(21) Application number: 25305456.3
(22) Date of filing: 28.03.2025
(51) Int. Cl.: A61B 34/10, A61B 17/16, A61B 34/20, A61B 34/30, A61B 90/00

(54) **DEVICE AND METHOD FOR DETERMINING A CORRECTED TRANSFORMATION FOR A SURGICAL TOOL**

(71) Applicant: Ecential Robotics, 38610 Gieres (FR)
(72) Inventor: LETZ, Olivier, 38610 Gières (FR); BARRUET, Rudy, 38610 Gières (FR)
(74) Representative: Icosa

(57) **Abstract**

The present invention relates to a first device (1) and method for providing an updated instructed transformation (31). The invention further relates to a second and third device and associated method for determining a corrected transformation for a surgical tool, based on an updated instructed transformation (31) obtained using the first device (1).

## Description

### FIELD OF INVENTION

The present invention relates to the technical field of computer-assisted surgery. More precisely, the invention relates to a first device and method for providing an updated instructed transformation. The invention further relates to a second device and a third device and associated methods for determining a corrected transformation for a surgical tool, based on an updated instructed transformation obtained using the first device.

### BACKGROUND OF INVENTION

In recent years, computer-assisted surgeries have significantly enhanced the precision and control available to surgeons. One key aspect of these systems is the ability to assist in calculating and guiding the trajectory of surgical tools during operations, ensuring that procedures are performed with minimal error. In many cases, a virtual boundary delineates a target surgical area. This virtual boundary is a predefined limit that the surgical tool should not cross during the procedure. Its purpose is to prevent damage to sensitive anatomical structures surrounding the surgical site and to ensure accurate cuts and movements within the prescribed area.

Existing technologies in the field employ real-time monitoring of the surgical tool's position relative to the virtual boundary. When the system detects that the surgical tool is about to cross or has crossed the virtual boundary, it automatically stops the surgical tool's movement to prevent unintended harm. While this method provides an essential safety feature, it introduces certain disadvantages.

A common issue with the current approach is the jerkiness in the movement of the surgical tool when it approaches the virtual boundary. The abrupt stopping of the surgical tool upon detection of a boundary crossing can result in saw-like or "see-saw" cuts along the tissue, which may compromise the smoothness and precision of the operation. This jerky motion can affect the quality of the surgical outcome, particularly in delicate procedures requiring precise, continuous movements.

Thus, the technical problem to be solved is how to achieve a smooth displacement of the surgical tool when approaching and interacting with a virtual boundary.

### SUMMARY

The invention advantageously aims at correcting the trajectory of a surgical tool to prevent it from crossing a virtual boundary. To that end, an adapted trajectory that ensures the surgical tool moves along the virtual boundary without crossing it is calculated. This approach results in a smooth displacement for the surgical tool, effectively minimizing or eliminating "see-saw" cuts and ensuring greater precision during surgical procedures. The invention further enables the virtual boundary to be used as a cutting guide, allowing the surgical tool to follow the virtual boundary smoothly, thereby ensuring precise and controlled incisions. This dual functionality ensures both safety and precision, making the invention particularly valuable for procedures requiring high accuracy, where inadvertent crossing of the virtual boundary or imprecise cutting could result in damage to critical tissues.

The invention notably finds an application during computer-assisted surgery to ensure the surgical tool moves along a virtual boundary that delineates a target anatomical structure to be operated on.

Such target anatomical structure may be a bone structure, cartilage, muscles, tendons, ligaments, or blood vessels. Notably, the target anatomical structure may be a bone structure destined to be cut or trimmed.

A surgical tool according to the invention may be a bone saw, a drill, a chisel, or a burr (e.g., a rotative burr).

For instance, a burr may be used during the placement of joint implants, such as hip or knee prostheses, to sculpt the bone and prepare the area to receive the implant. It can also be employed in the treatment of bone fractures to smooth or shape bone fragments or prepare the site for plates or screws. Additionally, in spinal surgery, a burr is often used to remove bone overgrowths (such as osteophytes) or to perform laminectomies, which involve removing part of the vertebrae to relieve spinal cord compression.

As another example, a bone saw may be used during total or partial knee replacement (e.g., unicompartmental prosthesis) to make precise cuts in the tibia and femur. It may also be employed during total hip replacement to prepare the acetabular cavity or to perform femoral cuts, such as removing the femoral head and neck. In addition, a bone saw can be utilized for corrective osteotomies to realign bones, for laminectomies to remove part of a vertebra to relieve spinal cord pressure, for preparing vertebrae for the placement of implants such as plates or rods, and for revision surgeries to carefully remove an old implant and reshape the bone to fit a new implant.

The above advantages may be obtained through a first device for providing an updated instructed transformation for preventing a first object from crossing a second object, wherein said updated instructed transformation is configured to be used to ensure controlled interaction between a surgical tool and a virtual boundary that delineates a target surgical area, such that the virtual boundary is respected and/or integrated into a trajectory of the surgical tool, said first device comprising:
- at least one input configured to receive:
   o current coordinates of vertices of said first object, said vertices being in an initial position, at least two of said vertices being connected by an edge to form said first object;
   o current coordinates of vertices of said second object, at least two of said vertices being connected by an edge to form said second object;
   o an instructed transformation to be applied to said vertices of said first object to displace the vertices from the initial position to an instructed position, said instructed transformation defining for each vertex of said first object, an associated instructed displacement vector between said initial position of said vertex and an instructed position of said vertex;
- at least one processor configured to:
   o for each vertex of the first object:
      ▪ calculate, using the current coordinates of the initial position of said vertex and the coordinates of vertices of said second object, at least one distance between the initial position of said vertex and an intersection point with at least one edge of the second object along a direction of the instructed displacement vector associated with said vertex,
      ▪ compute a result of a function of said calculated at least one distance,
   o select a vertex of reference based on said computed results,
   o determine an orthogonal projection point of an instructed position of said vertex of reference on said at least one edge of the second object comprising the intersection point,
   o update the instructed transformation using said determined orthogonal projection point and said result of the function of distance obtained for the vertex of reference,
- at least one output configured to provide said updated instructed transformation.

More precisely, the first device described above is configured to receive as input two objects, each defined by the current coordinates of their vertices, and the intended trajectory (i.e., the instructed transformation) of one object with respect to the other. Notably, the first object and/or the second object may be a polygon. The first device may for instance be configured to receive current coordinates of control points associated to predefined locations on the surgical tool, wherein the control points are connected by edges to form a representation of the surgical tool (e.g. a polygonal representation of the surgical tool or a polyline if the representation of the surgical tool is a connected series of edges but does not necessarily form a closed shape) and current coordinates of boundary points forming a representation of the virtual boundary when connected by boundary segments (e.g. a polygonal representation of the virtual boundary or a polyline if the virtual boundary is a connected series of boundary segments but does not necessarily form a closed shape).

Based on these inputs, the first device selects a vertex of reference among the vertices of the first object. The vertex of reference is typically selected as the first vertex of one object that is expected to intersect with the other object during their respective trajectories.

An updated instructed transformation is then calculated using the selected vertex of reference, to ensure that one object does not cross the other object. The updated instructed transformation is notably obtained using orthogonal projection, which is well suited for complex polygonal shapes, as it can identify precise relationships between vertices and edges without requiring overly complex calculations.

Several functions of distance may be implemented within the first object.

According to one embodiment, the function of said calculated at least one distance computed for each vertex of the first object is a ratio between the at least one distance calculated for each vertex and a magnitude of the instructed displacement vector associated with each vertex.

According to one embodiment, the function of said calculated at least one distance computed for each vertex of the first object is the identity function.

In the same manner, several solutions may be implemented to select the vertex of reference within the first device.

According to one embodiment, the vertex of reference corresponds to the vertex of the first object associated with the smallest computed result.

Thus, according to one embodiment, the vertex of reference corresponds to the vertex of the first object associated with the smallest distance when the function of said calculated at least one distance computed for each vertex of the first object is the identity function.

According to another embodiment, the vertex of reference corresponds to the vertex of the first object associated with the smallest ratio when the function of said calculated at least one distance computed for each vertex of the first object is a ratio between the at least one distance calculated for said vertex and a magnitude of the instructed displacement vector associated with said vertex.

Regarding the instructed transformation received as input by the first device, several embodiments are possible.

According to one embodiment, the instructed transformation comprises only a translation component. In other words, the instructed transformation does not comprise a rotation component.

According to another embodiment, the instructed transformation comprises a translation component and a rotation component.

In the same manner, according to one embodiment, the updated instructed transformation comprises only a translation component. In other words, the updated instructed transformation does not comprise a rotation component.

According to another embodiment, the updated instructed transformation comprises a translation component and a rotation component.

The components may be expressed in a single matrix (i.e., one matrix with a translation component and a rotation component) or in at least two matrices (e.g., one matrix comprising the translation component and one matrix comprising the rotation component).

According to one embodiment, the translation component and the rotation component of the instructed transformation are updated independently to obtain the updated instructed transformation.

For instance, according to one embodiment, the translation component is updated using the orthogonal projection point coordinates.

According to another embodiment, the rotation component is updated using the result of the function of distance.

Notably, according to one embodiment, the rotation component is updated by multiplying the rotation component by the result of the function of distance.

According to an embodiment, when the instructed displacement vector associated with the vertex of reference intersects a vertex of the second object, said vertex of the second object being an intersection between two edges of the second object, including a first edge and a second edge, the instructed displacement vector forming a first angle with the first edge and the instructed displacement vector forming a second angle with the second edge, said at least one processor is further configured to:
- select, between the first edge and the second edge, an edge forming a smallest angle with the instructed displacement vector, between the first angle and the second angle, and
- calculate the at least one distance using the selected edge.

Two main scenarios wherein the representation of the surgical tool can cross the representation of the virtual boundary may be identified.

In the first scenario, the first vertex of one object that is expected to intersect with the other object is a vertex of the representation of the surgical tool. In other words, during the movements of the representation of the surgical tool, one of its vertices will intersect the representation of the virtual boundary (rather than along one of its edges). In that case, at least one vertex of the representation of the surgical tool is localized inside the representation of the virtual boundary.

Therefore, another aspect of the invention pertains to a second device for determining a corrected transformation for a surgical tool, based on an updated instructed transformation obtained using the first device, said second device comprising:
- at least one input configured to receive a current displacement instruction comprising:
   o current coordinates of control points corresponding to an initial position of said surgical tool, wherein said control points are associated to predefined locations on the surgical tool and at least two control points are connected by an edge to form a representation of the surgical tool;
   o current coordinates of boundary points, said boundary points delimitating a virtual boundary, at least two boundary points being connected by a boundary segment to form a representation of the virtual boundary;
   o an initial transformation to be applied to the current coordinates of control points,
- at least one processor configured to:
   o apply the initial transformation to said control points, so as to obtain a displaced representation of said surgical tool,
   o determine if the displaced representation of the surgical tool intersects the representation of the virtual boundary:
      ▪ if said displaced representation of the surgical tool does not intersect said representation of the virtual boundary, define said corrected transformation as said initial transformation,
      ▪ if said displaced representation of the surgical tool does intersect the representation of the virtual boundary:
         - provide as an input to the first device said current coordinates of control points as current coordinates of vertices of said first object, said current coordinates of boundary points as current coordinates of vertices of said second object, and said initial transformation as instructed transformation, so that the first object corresponds to the representation of the surgical tool and the second object corresponds to the representation of the virtual boundary;
         - obtain as an output an updated instructed transformation,
- at least one output configured to provide said updated instructed transformation as the corrected transformation.

In other words, the second device receives as input a representation of the surgical tool defined by the current coordinates of its control points and a representation of the virtual boundary defined by the current coordinates of its boundary points. Additionally, the second device receives the initially intended trajectory of the surgical tool (e.g. initial transformation to be applied to the current coordinates of control points).

This initially intended trajectory may be provided manually by a user operating a robotic arm on which the surgical tool is mounted or automatically calculated by a robotic arm equipped with a surgical tool, based on pre-defined surgical plans or intraoperative adjustments.

The second device is configured to feed these input data to the first device so that the first device may select a vertex of reference of the representation of the surgical tool (e.g., the first control point expected to cross the representation of the virtual boundary) and calculate an updated instructed transformation that does not cross the representation of the virtual boundary.

The second device is then configured to update the initially intended trajectory (e.g. initial transformation) of the surgical tool to a corrected trajectory (e.g. corrected transformation) that does not cross the representation of virtual boundary.

In the second scenario, the first vertex of one object that is expected to intersect with the other object is a vertex of the representation of the virtual boundary. In other words, during the movements of the representation of the surgical tool, one of its edges will intersect the virtual boundary but none of its vertices will cross the virtual boundary.

Therefore, another aspect of the invention pertains to a third device for determining a corrected transformation for a surgical tool, based on an updated instructed transformation obtained using the first device, said third device comprising:
- at least one input configured to receive a current displacement instruction comprising:
   o current coordinates of control points corresponding to an initial position of said surgical tool, wherein said control points are associated to predefined locations on the surgical tool and at least two control points are connected by an edge to form a representation of the surgical tool;
   o current coordinates of boundary points, said boundary points delimitating a virtual boundary, at least two boundary points being connected by a boundary segment to form a representation of the virtual boundary;
   o an initial transformation to be applied to the current coordinates of control points,
- at least one processor configured to:
   o apply the initial transformation to said control points so as to obtain a displaced representation of said surgical tool,
   o determine if the displaced representation of the surgical tool intersects the representation of the virtual boundary:
      ▪ if said displaced representation of the surgical tool does not intersect said representation of the virtual boundary, define said corrected transformation as said initial transformation,
      ▪ if said projected representation of the surgical tool does intersect the representation of the virtual boundary:
         - calculate an inverse transformation of the initial transformation,
         - provide as an input to the first device said current coordinates of boundary points as current coordinates of vertices of said first object, said current coordinates of control points as current coordinates of vertices of said second object, and said inverse transformations as instructed transformation, so that the first object corresponds to the representation of the virtual boundary and the second object corresponds to the representation of the surgical tool,
         - obtain as an output an updated instructed transformation,
         - calculate an inverse updated transformation using the updated instructed transformation,
- at least one output configured to provide said inverse updated transformation as said corrected transformation.

In other words, the third device receives as input a representation of the surgical tool defined by the current coordinates of its control points and a representation of the virtual boundary defined by the current coordinates of its boundary points. Additionally, the third device receives the initially intended trajectory of the surgical tool (e.g. initial transformation to be applied to the current coordinates of control points).

This initially intended trajectory may be provided manually by a user operating a robotic arm on which the surgical tool is mounted or automatically calculated by a robotic arm equipped with a surgical tool, based on pre-defined surgical plans or intraoperative adjustments.

The third device is configured to feed to the first device the current coordinates of the control points and the coordinates of the boundary points received as input, together with a transformation to be applied to the representation of the virtual boundary. This transformation corresponds to an inverse transformation of the initial transformation to be applied to the control points.

In other words, in this scenario, the first device is used to virtually manipulate the representation of the virtual boundary, although in reality the virtual boundary itself does not physically move. This allows the device to determine which vertex (i.e., vertex of reference) of the representation of the virtual boundary intersects with the representation of the surgical tool. Based on this, the first device calculates an updated instructed transformation configured to ensure that the trajectory of the representation of the virtual boundary does not cross the representation of the surgical tool.

The third device is then configured to update the initially intended trajectory (e.g. initial transformation) of the surgical tool to a corrected trajectory (e.g. corrected transformation) that does not cross the virtual boundary.

According to other aspects of the invention, the second device and/or the third device comprise one or more of the features described in the following embodiments, taken alone or in any possible combination.

To ensure continuous and precise operation of the second device and/or the third device within the defined virtual boundary, new current displacement instructions are periodically processed. For example, this occurs when a user manipulates the robotic arm to initiate movement or when the robotic arm initiates an automated motion. Specifically, each time the second device and/or the third device receives a new current displacement, the at least one processor evaluates whether it would cause the surgical to cross the virtual boundary. A new current displacement instruction may comprise new current coordinates of control points corresponding to a new initial position of the surgical tool and/or new current coordinates of boundary points delimitating the virtual boundary.

Therefore, according to an embodiment, the at least one input of the second device and/or the third device is configured to periodically receive a new current displacement instruction, said at least one processor being configured to calculate a corrected transformation each time a new current displacement instruction is received.

The invention further encompasses several embodiments that define and manage virtual boundaries.

According to one embodiment, the virtual boundary is defined preoperatively (i.e., established prior to the surgical procedure). In this embodiment, the current coordinates of boundary points received as input by the second device and/or third device remain unchanged over time (e.g., during the whole surgical procedure).

According to another embodiment, the virtual boundary may be redefined intraoperatively, for example by the user. In this embodiment, the current coordinates of boundary points received as input by the second device and/or third device are updated dynamically during the surgical procedure. New current coordinates of boundary points are received each time the virtual boundary is redefined. This allows to adapt the virtual boundary in real-time based on the needs of the surgical procedure, offering greater flexibility and control over the constrained movement of the surgical tool. The virtual boundary may for instance be modified to constrain the movement of the surgical tool within a specific area of anatomical interest that is newly defined during the surgical procedure (e.g., adjustments made in response to newly discovered conditions or unforeseen findings during the surgical procedure).

Thus, according to this embodiment, the at least one input of the second device and/or third device is configured to periodically receive current coordinates of boundary points to redefine the virtual boundary, said at least one processor being configured to calculate a corrected transformation each time current coordinates of boundary points are received.

According to one embodiment, safety measures may be implemented to address scenarios where the surgical tool finds itself outside the redefined boundary. Safety measures may include at least one of the following: the system may halt the robotic arm movements, the system may emit a warning signal, which could be luminous or audible. Alternatively, the redefinition of the virtual boundary could be restricted, requiring the new virtual boundary to include the surgical tool within itself. Another option is to automatically reposition the surgical tool to comply with the redefined boundary.

Thus, according to this embodiment, if the projected representation of the surgical tool is determined to be inside the representation of the virtual boundary, the at least one processor is configured to implement a safety measure to prevent further deviation and restore compliance with the virtual boundary.

To determine whether the representation of the surgical tool is inside the representation of the virtual boundary, the at least one processor may implement a point-in-polygon algorithm (e.g., ray-casting or winding number).

According to another embodiment, a second virtual boundary may be defined by the movement of the surgical tool within a first virtual boundary predefined preoperatively. In this embodiment, the second virtual boundary may be dynamically defined as the surgical tool moves, thereby marking an area that has been cut and separating it from a remaining area to be cut. This dynamic boundary aids in tracking the progress of the procedure and ensures precise execution within the predefined area.

According to one embodiment, the virtual boundary or the surgical tool's movements may be adapted based on specific surgical parameters. For instance, adjustments can be made depending on the number of passes the instrument has made within the cutting area, the rotational speed of the motor, or the amount of material remaining to be cut. This adaptive approach allows the system to optimize the surgical tool movements, ensuring smooth navigation along the virtual boundary and preventing excessive material removal when the motor speed is insufficient to handle a larger workload.

Another aspect of the invention relates to a first ensemble comprising the second device and the third device, wherein said first ensemble comprises:
- at least one processor communicatively coupled to the second device and the third device, said at least one processor being configured to send an execution request to either one of the second device and the third device so as to obtain as output a first corrected transformation, said at least one processor being further configured to provide said first corrected transformation as the initial transformation of the other of the two devices, between the second device and the third device, so as to obtain a second corrected transformation,
- at least one output configured to provide said corrected transformation.

In other words, the first ensemble is configured to successively check the two scenarios mentioned above (i.e. first scenario: during the movements of the representation of the surgical tool, one of its vertices intersects the representation of the virtual boundary, and second scenario: during the movements of the polygonal representation of the surgical tool, one of its edges intersects the virtual boundary but none of its vertices crosses the virtual boundary). This comprehensive approach allows for a more thorough verification, minimizing the risk of overlooking potential breaches of the virtual boundary.

Another aspect of the invention relates to a second ensemble comprising the second device and the third device, wherein said ensemble comprises:
- at least one processor communicatively coupled to the second device and the third device, said at least one processor being configured to send an execution request to the second device and the third device so as to obtain a third corrected transformation as output of the second device and a fourth corrected transformation as output of the third device, said at least one processor being further configured to select a corrected transformation between the third corrected transformation and the fourth corrected transformation with respect to a criterion, wherein said criterion corresponds to a minimization of the update to the initial transformation, and
- at least one output configured to provide the selected corrected transformation.

According to the invention, a minimization of the update made to a transformation refers to the process of selecting the transformation that results in the smallest modification when compared to the initial transformation. This can be quantitatively defined based on a chosen metric, such as the difference in translation, rotation, or a combination of both. The difference may be computed using the Euclidean distance for translation components, the angular difference (e.g., quaternion or rotation matrix deviation) for rotational components or a weighted combination of the Euclidean distance and angular difference. The transformation with the smallest calculated difference may be selected.

According to other aspects of the invention, the first ensemble and/or second ensemble comprise one or more of the features described in the following embodiments, taken alone or in any possible combination.

According to one embodiment, the at least one input of the first ensemble and/or second ensemble is configured to periodically receive a new current displacement instruction, said at least one processor being configured to calculate a corrected transformation each time a new current displacement instruction is received.

According to one embodiment, if the displaced representation of the control points is determined to intersect the representation of the virtual boundary, the at least one processor is configured to implement a safety measure to prevent further deviation and restore compliance with the representation of the virtual boundary.

Another aspect of the invention relates to a surgical system comprising:
- a robotic arm configured to control a trajectory of at least one surgical tool, said robotic arm comprising at least one processor configured to receive a movement instruction initiated by a user manipulating the robotic arm, said at least one processor being configured to determine said initial transformation based on said received movement instruction,
- the first ensemble and/or second ensemble, wherein said first ensemble and/or second ensemble is configured to receive as input the initial transformation determined by said at least one processor and to output a corrected transformation, said at least one processor being configured to implement the corrected transformation to control movements of the robotic arm so as to maintain the surgical tool within the virtual boundary.

Another aspect of the invention relates to a first method for providing an updated instructed transformation for preventing a first object from crossing a second object, wherein said updated instructed transformation is configured to be used to ensure controlled interaction between a surgical tool and a virtual boundary that delineates a target surgical area, such that the virtual boundary is respected and/or integrated into a trajectory of the surgical tool, said first method comprising:
- receiving:
   o current coordinates of vertices of said first object, said vertices being in an initial position, at least two of said vertices being connected by an edge to form said first object;
   o current coordinates of vertices of said second object, at least two of said vertices being connected by an edge to form said second object;
   o an instructed transformation to be applied to said vertices of said first object to displace the vertices from the initial position to an instructed position, said instructed transformation defining for each vertex of said first object, an associated instructed displacement vector between said initial position of said vertex and an instructed position of said vertex;
- for each vertex of the first object:
   o calculating, using the current coordinates of the initial position of said vertex and the coordinates of vertices of said second object, at least one distance between the initial position of said vertex and an intersection point with at least one edge of the second object along a direction of the instructed displacement vector associated with said vertex,
   o computing a result of a function of said calculated at least one distance,
- selecting a vertex of reference based on said computed results,
- determining an orthogonal projection point of an instructed position of said vertex of reference on said at least one edge of the second object comprising the intersection point,
- updating the instructed transformation using said determined orthogonal projection point and said result of the function of distance obtained for the vertex of reference,
- providing said updated instructed transformation.

The invention further relates to a device for determining a corrected transformation for a surgical tool, based on an updated instructed transformation obtained using the first method described above, said device comprising:
- at least one input configured to receive a current displacement instruction comprising:
   o current coordinates of control points corresponding to an initial position of said surgical tool, wherein said control points are associated to predefined locations on the surgical tool and at least two control points are connected by an edge to form a representation of the surgical tool;
   o current coordinates of boundary points, said boundary points delimitating a virtual boundary, at least two boundary points being connected by a boundary segment to form a representation of the virtual boundary;
   o an initial transformation to be applied to the current coordinates of control points,
- at least one processor configured to:
   o apply the initial transformation to said control points, so as to obtain a displaced representation of said surgical tool,
   o determine if the displaced representation of the surgical tool intersects the representation of the virtual boundary:
      ▪ if said displaced representation of the surgical tool does not intersect said representation of the virtual boundary, define said corrected transformation as said initial transformation,
      ▪ if said displaced representation of the surgical tool does intersect the representation of the virtual boundary:
         - implement the first method using as input said current coordinates of control points as current coordinates of vertices of said first object, said current coordinates of boundary points as current coordinates of vertices of said second object, and said initial transformation as instructed transformation, so that the first object corresponds to the representation of the surgical tool and the second object corresponds to the representation of the virtual boundary;
         - obtain as an output said updated instructed transformation,
- at least one output configured to provide said updated instructed transformation as the corrected transformation.

The invention further relates to a device for determining a corrected transformation for a surgical tool, based on an updated instructed transformation obtained using the first method described above, said device comprising:
- at least one input configured to receive a current displacement instruction comprising:
- least one input configured to receive a current displacement instruction comprising:
   o current coordinates of control points corresponding to an initial position of said surgical tool, wherein said control points are associated to predefined locations on the surgical tool and at least two control points are connected by an edge to form a representation of the surgical tool;
   o current coordinates of boundary points, said boundary points delimitating a virtual boundary, at least two boundary points being connected by a boundary segment to form a representation of the virtual boundary;
   o an initial transformation to be applied to the current coordinates of control points;
- at least one processor configured to:
   o apply the initial transformation to said control points so as to obtain a displaced representation of said surgical tool,
   o determine if the displaced representation of the surgical tool intersects the representation of the virtual boundary:
      ▪ if said displaced representation of the surgical tool does not intersect said representation of the virtual boundary, define said corrected transformation as said initial transformation,
      ▪ if said projected representation of the surgical tool does intersect the representation of the virtual boundary:
         - calculate an inverse transformation of the initial transformation,
         - implement the first method using said current coordinates of boundary points as current coordinates of vertices of said first object, said current coordinates of control points as current coordinates of vertices of said second object, and said inverse transformations as instructed transformation, so that the first object corresponds to the representation of the virtual boundary and the second object corresponds to the representation of the surgical tool,
         - obtain as an output an updated instructed transformation,
         - calculate an inverse updated transformation using the updated instructed transformation,
- at least one output configured to provide said inverse updated transformation as said corrected transformation.

Another aspect of the invention relates to a second method for determining a corrected transformation for a surgical tool, based on an updated transformation of reference obtained using the first method, said second method comprising:
- receiving a current displacement instruction comprising:
   o current coordinates of control points corresponding to an initial position of said surgical tool, wherein said control points are associated to predefined locations on the surgical tool and at least two control points are connected by an edge to form a representation of the surgical tool;
   o current coordinates of boundary points, said boundary points delimitating a virtual boundary, at least two boundary points being connected by a boundary segment to form a representation of the virtual boundary;
   o an initial transformation to be applied to the current coordinates of control points;
- applying said initial transformation to said control points, so as to obtain a displaced representation of said surgical tool,
- determining if the displaced representation of the surgical tool intersects the representation of the virtual boundary:
   o if said displaced representation of the surgical tool does not intersect said representation of the virtual boundary, define said corrected transformation as said initial transformation,
   o if said displaced representation of the surgical tool does intersect the representation of the virtual boundary:
      o implementing the first method using said current coordinates of control points as current coordinates of vertices of said first object, said current coordinates of boundary points as current coordinates of vertices of said second object, and said initial transformation as instructed transformation, so that the first object corresponds to the representation of the surgical tool and the second object corresponds to the representation of the virtual boundary,
      ∘ obtain as an output an updated instructed transformation,
- providing said updated instructed transformation as the corrected transformation.

Another aspect of the invention relates to a third method for determining a corrected transformation for a surgical tool, based on an updated instructed transformation obtained using the first method described above, said third method comprising:
- receiving a current displacement instruction comprising:
   o current coordinates of control points corresponding to an initial position of said surgical tool, wherein said control points are associated to predefined locations on the surgical tool and at least two control points are connected by an edge to form a representation of the surgical tool;
   o current coordinates of boundary points, said boundary points delimitating a virtual boundary, at least two boundary points being connected by a boundary segment to form a representation of the virtual boundary;
   o an initial transformation to be applied to the current coordinates of control points;
- applying the initial transformation to said control points so as to obtain a displaced representation of said surgical tool,
- determining if the displaced representation of the surgical tool intersects the representation of the virtual boundary:
   o if said displaced representation of the surgical tool does not intersect said representation of the virtual boundary, define said corrected transformation as said initial transformation,
   o if said projected representation of the surgical tool does intersect the representation of the virtual boundary:
      ▪ calculating an inverse transformation of the initial transformation,
      ▪ implementing the first method using said current coordinates of boundary points as current coordinates of vertices of said first object, said current coordinates of control points as current coordinates of vertices of said second object, and said inverse transformations as instructed transformation, so that the first object corresponds to the representation of the virtual boundary and the second object corresponds to the representation of the surgical tool,
      ▪ obtaining as an output an updated instructed transformation,
      ▪ calculating an inverse updated transformation using the updated instructed transformation,
- providing said inverse updated transformation as said corrected transformation.

In addition, the disclosure relates to a computer program product comprising instructions which, when the program is executed by a computer, cause the computer to carry out at least one of the methods described above.

The present disclosure further pertains to a computer-readable storage medium comprising instructions which, when executed by a computer, cause the computer to carry out at least one of the methods described above.

The present disclosure further pertains to a non-transitory program storage device, readable by a computer, tangibly embodying a program of instructions executable by the computer to perform at least one of the methods compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:

The terms "adapted" and "configured" are used in the present disclosure as broadly encompassing initial configuration, later adaptation or complementation of the present device, or any combination thereof alike, whether effected through material or software means (including firmware).

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The above definitions are compliant with their usual meaning, and can be completed with numerous associated features and properties, and definitions of related numerical objects, well known to a person skilled in the ML field. Additional terms will be defined, specified or commented wherever useful throughout the following description.

### BRIEF DESCRIPTION OF THE DRAWINGS

**Figure 1** is a block diagram representing schematically a particular mode of a first device for providing an updated instructed transformation, compliant with the present disclosure;
**Figure 2** is a flow chart showing successive steps executed with the first device for providing an updated instructed transformation of figure 1;
**Figure 3** is a block diagram representing schematically a particular mode of a second device for determining a corrected transformation, based on an updated instructed transformation obtained using the first device from figure 1, compliant with the present disclosure;
**Figure 4** is a flow chart showing successive steps executed with the second device for determining a corrected transformation of figure 3;
**Figure 5** is a block diagram representing schematically a particular mode of a third device for determining a corrected transformation, based on an updated instructed transformation obtained using the first device from figure 1, compliant with the present disclosure;
**Figure 6** is a flow chart showing successive steps executed with the third device for determining a corrected transformation of figure 5;
**Figure 7** is a block diagram representing schematically the interactions between the second device from figure 3 and the first device from figure 1;
**Figure 8** is a block diagram representing schematically the interactions between the third device from figure 5 and the first device from figure 1;
**Figure 9** is a schematic view of a representation of the surgical tool and a representation of the virtual boundary, wherein the representation of the surgical tool is in an initial position;
**Figure 10** is a schematic view of a representation of the surgical tool and a representation of the virtual boundary, wherein the representation of the surgical tool is projected in the instructed position;
**Figure 11** is a schematic view of a representation of the surgical tool and a representation of the virtual boundary, wherein the control point of reference is selected;
**Figure 12** is a schematic view of a representation of the surgical tool and a representation of the virtual boundary, wherein an orthogonal projection of the projected control point is made on the representation of the virtual boundary;
**Figure 13** is a schematic view of a representation of the surgical tool and a representation of the virtual boundary, wherein an updated instructed transformation is defined;
**Figure 14** is a schematic view of a representation of the surgical tool and a representation of the virtual boundary, wherein the representation of the surgical tool is in a corrected position determined using the updated instructed transformation;
**Figure 15** is a schematic view of a representation of the surgical tool and a representation of the virtual boundary, wherein the representation of the surgical tool is represented in successive corrected positions;
**Figure 16** is a schematic view of a representation of the surgical tool and a representation of the virtual boundary, wherein the representation of the surgical tool is in an initial position;
**Figure 17** is a schematic view of a representation of the surgical tool and a representation of the virtual boundary, wherein the representation of the surgical tool is projected in the instructed position;
**Figure 18** is a schematic view of a representation of the surgical tool and a representation of the virtual boundary, wherein opposite displacement vectors are projected from boundary point onto the representation of the surgical tool;
**Figure 19** is a schematic view of a representation of the surgical tool and a representation of the virtual boundary, wherein the boundary point of reference is selected;
**Figure 20** is a schematic view of a representation of the surgical tool and a representation of the virtual boundary, wherein an orthogonal projection of the projected boundary point is made on the representation of the surgical tool;
**Figure 21** is a schematic view of a representation of the surgical tool and a representation of the virtual boundary, wherein an updated instructed transformation is defined;
**Figure 22** is a schematic view of a representation of the surgical tool and a representation of the virtual boundary, wherein the representation of the surgical tool is in a corrected position obtained using the updated instructed transformation;
**Figure 23** is a schematic view of a representation of the surgical tool and a representation of the virtual boundary, wherein the instructed displacement vector associated with the control point of reference crosses a boundary point; and
**Figure 24** illustrates an apparatus embodying the devices compliant with the present disclosure, such as the devices of Figure 1, Figure 3 and Figure 5.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

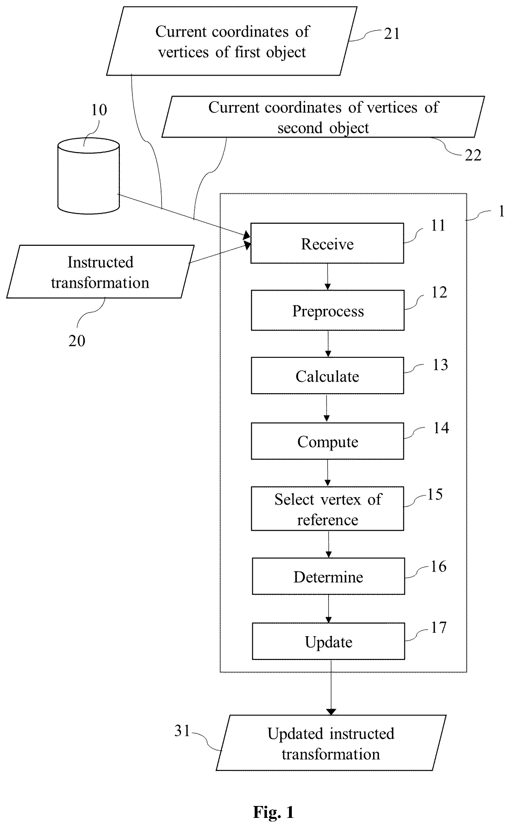

The present disclosure will be described in reference to a particular functional embodiment of a device 1 for providing an updated instructed transformation 31 for preventing a first object from crossing a second object, as illustrated on **Figure 1****.**

The device 1 is adapted to receive as input current coordinates of vertices of a first object 21, current coordinates of vertices of a second object 22 and an instructed transformation 20 to be applied to the vertices of the first object to displace it from an initial position to an instructed position.

The device 1 is configured to output an updated instructed transformation 31.

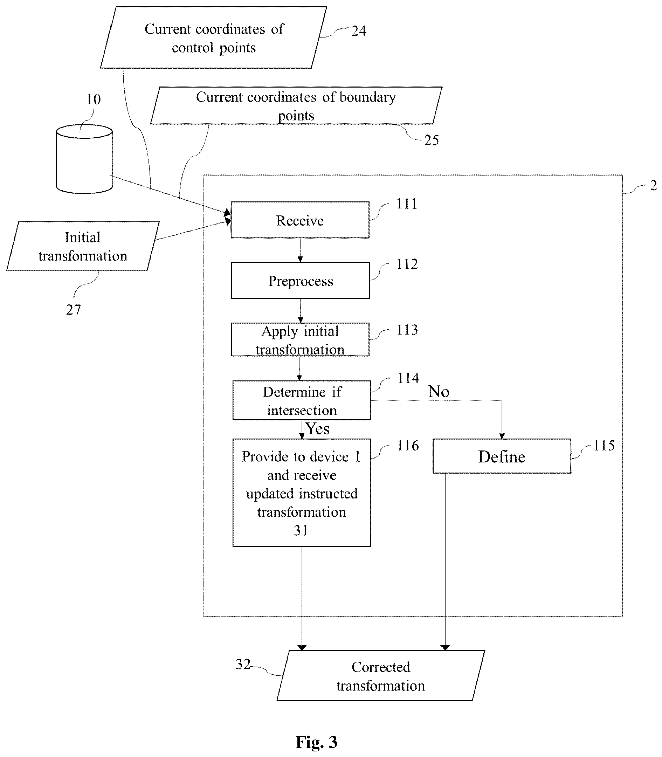

The device 1 for providing an updated instructed transformation 31 is associated with a device 2, represented on **Figure 3****,** for determining a corrected transformation 32 for a surgical tool, based on the updated instructed transformation 31 obtained using the device 1.

The device 2 is adapted to receive as input current coordinates of control points 24 of the surgical tool, current coordinates of boundary points 25 and an initial transformation 27 to be applied to the current coordinates of control points 24.

The device 2 is configured to output a corrected transformation 32. To that end, the device 2 is configured to provide as an input to the device 1, the current coordinates of control points 24, the current coordinates of boundary points 25 and the initial transformation 27, so as to obtain an updated instructed transformation 31 corresponding to the corrected transformation 32 to output.

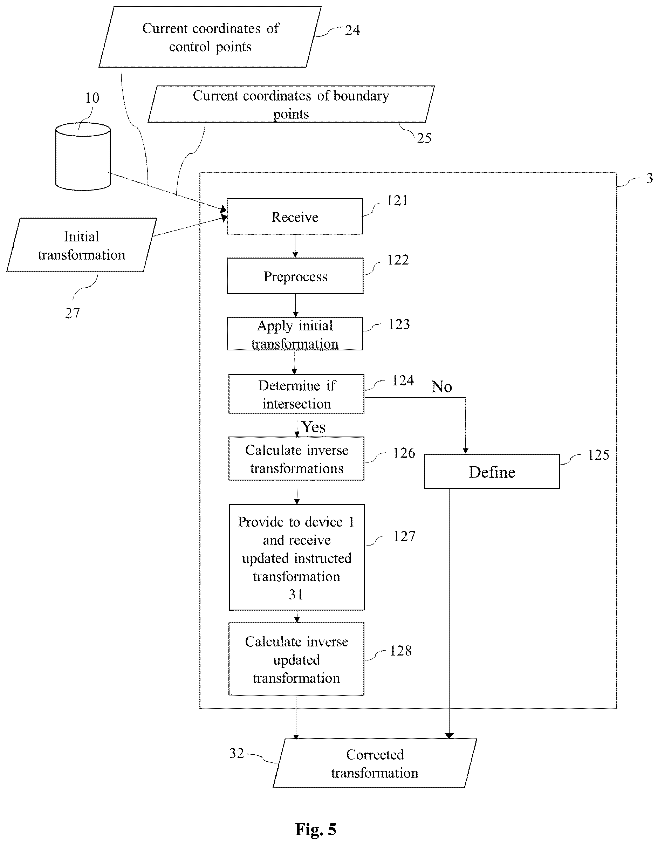

The device 1 for providing an updated instructed transformation 31 is further associated with a device 3, represented on **Figure 5****,** for determining a corrected transformation 32 for a surgical tool, based on the updated instructed transformation 31 obtained using the device 1.

The device 3 is adapted to receive as input current coordinates of control points 24, current coordinates of boundary points 25 and an initial transformation 27 to be applied to the current coordinates of control points 24.

The device 3 is configured to output a corrected transformation 32. To that end, the device 3 is configured to provide as an input to the device 1, the current coordinates of control points 24, the current coordinates of boundary points 25 and an inverse transformation 78 calculated using the initial transformation 27, so as to obtain an updated instructed transformation 31. The corrected transformation 32 is then determined, based on an inverse updated instructed transformation 79, calculated using the updated instructed transformation 31 obtained from device 1.

The devices 1, 2, and 3 are designed for use during computer-assisted surgeries to ensure precise control of surgical tools. To that end, devices 1, 2, and 3 may be integrated within a robotic arm designed to work alongside and/or to be guided by the surgeon. In such a setup, the robotic arm assists the surgeon by providing enhanced precision, stability, and the ability to smoothly follow along predefined boundaries during complex surgical tasks.

A person skilled in the art, without necessarily having access to the underlying algorithm, would be able to identify that the invention is implemented by observing the cut performed by the surgical tool and the responsiveness of the robotic arm.

In the absence of the invention, when a surgical tool moves along a virtual boundary, the user is forced to manually adjust the joystick or reposition the instrument after it makes contact with the boundary and is automatically halted. This process typically involves a frustrating trial-and-error approach, where the user must repeatedly guess the correct joystick command or angle to continue the movement, resulting in a cumbersome and inefficient user experience. Moreover, the lack of automated control leads to inconsistent motion, producing a "see-saw" type cut, which is irregular and lacks the smoothness required for precise surgical procedures.

However, when the present invention is embedded into the robotic arm, a skilled person in the art can visually determine its presence and functionality. Specifically, the cut created by the surgical tool along the virtual boundary is smoothly contoured and continuous, indicating that the invention's algorithm is actively managing the surgical tool movements. The smoothness of the cut reflects the advanced control system, which eliminates the need for trial-and-error and allows the surgical tool to follow the virtual boundary seamlessly, improving user-friendliness and operational efficiency. As a result, the user experiences a more intuitive and efficient interaction with the system, where movements are smooth and uninterrupted, without abrupt stops, greatly enhancing user-friendliness and overall operational efficiency.

The devices 1, 2 and 3 can process displacement instructions in real-time, either immediately upon receiving a new displacement instruction or at regular intervals. These intervals can typically be every 40 milliseconds, to ensure continuous and smooth adjustments during the surgery.

Though the presently described devices 1, 2 and 3 are versatile and provided with several functions that can be carried out alternatively or in any cumulative way, other implementations within the scope of the present disclosure include devices having only parts of the present functionalities.

Each of the devices 1, 2 and 3 is advantageously an apparatus, or a physical part of an apparatus, designed, configured and/or adapted for performing the mentioned functions and produce the mentioned effects or results. In alternative implementations, any of the device 1, the device 2 and/or the device 3 is embodied as a set of apparatus or physical parts of apparatus, whether grouped in a same machine or in different, possibly remote, machines. The device 1 and/or the device 2 and/or the device 3 may have functions distributed over a cloud infrastructure and be available to users as a cloud-based service, or have remote functions accessible through an API.

The device 1, the device 2 and the device 3 may be integrated in a same apparatus or set of apparatus, and intended to same users. In other implementations, the structure of the device 2 may be completely independent of the structure of the device 1 and may be provided for other users. In the same manner, the structure of the device 3 may be completely independent of the structure of the device 1 and/or the structure of the device 2 may be completely independent from the structure of the device 3.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device 2 or of the device 3.

The device 1 may comprise a **module** 11 for receiving the current coordinates of vertices of the first object 21, the current coordinates of vertices of the second object 22 and the instructed transformation 20 to be applied to the vertices of the first object 21.

In the present context, "current" indicates that the coordinates of the first object 21 and the coordinates of the second object 22 may be continuously updated as the invention may be implemented in a dynamic and iterative manner. Each time a new instructed transformation 20 (e.g., instruction of movement) is received, the device 1 re-evaluates whether the first object intersects or crosses the second object. However, even in a single iteration, the invention provides significant value, as it allows for an immediate assessment of whether the first object 21 intersects or crosses the second object 22 based on the instructed transformation 20. This enables real-time decision-making and responsiveness, ensuring that even without continuous updates, the system can still deliver meaningful insights and operational benefits.

The current coordinates of vertices of the first object 21 and the current coordinates of vertices of the second object 22 may be 2D coordinates. They may be provided in various formats, depending on the application and data structure. For instance, the current coordinates may be cartesian coordinates or polar coordinates, represented in lists, arrays, or matrices or in text-based formats like CSV, JSON, or XML; or in geometric formats like SVG or CAD files. As a particular example, the current coordinates of the vertices of the first object 21 and the current coordinates of the vertices of the second object 22 may be expressed relative to the origin of a coordinate system, potentially using transformations that describe their positions and orientations in that system.

The instructed transformation 20 may be expressed as a transformation matrix configured to be applied to the vertices of the first object to compute the instructed position of the first object. The instructed transformation 20 may include a translation component. The instructed transformation 20 may further include a rotation component.

In some embodiments, the instructed transformation 20 may be represented as a single transformation matrix that integrates both translation and rotation components. In other embodiments, the instructed transformation 20 may be decomposed into multiple matrices, each corresponding to a distinct transformation operation. For instance, a first matrix may define a translation transformation, a second matrix may define a rotation transformation. These matrices may be applied sequentially, either in a predetermined order or based on specific transformation parameters, to compute the instructed position of the first object.

To facilitate understanding, the instructed transformation 20 is represented on the figures as instructed displacement vectors 65, 165. Notably, the instructed displacement vectors may be identical for all vertices of the first object 21 when the transformation consists solely of a translation. Alternatively, they may differ from one vertex to another to account for a rotation of the first object 21.

All these input data may be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

**Module 12** may be optionally configured to perform a preprocessing of the input data. For example, preprocessing may include filtering out redundant or irrelevant coordinates or normalizing the input data to a common reference frame.

The device 1 may also include a **module 13** configured to calculate the intersection points between the first object in its projected position (i.e., projected first object) and the second object. The intersection points may for instance comprise at least one intersection point or at least two intersection points. To that end, **module 13** may be configured to apply the instructed transformation 20 to the vertices of the first object so as to project the first object in its instructed position. This allows to obtain coordinates of the vertices of the projected first object, in its instructed position. The intersection points may be calculated by using the current coordinates of vertices of the second object 22 and the coordinates of the vertices of the projected first object. A point-in-polygon algorithm may for instance be implemented to determine the intersection points.

**Module 13** may be further configured, for each vertex of the first object, to calculate the distance between the vertex initial position and the at least one intersection point with the second object. The distance is generally measured along the instructed displacement vector 65 associated with the vertex (i.e., vector defined between the initial position of said vertex and the instructed position of said vertex). Therefore, the distance is generally measured between the vertex initial position and the intersection point located on the instructed displacement vector 65 associated with the vertex.

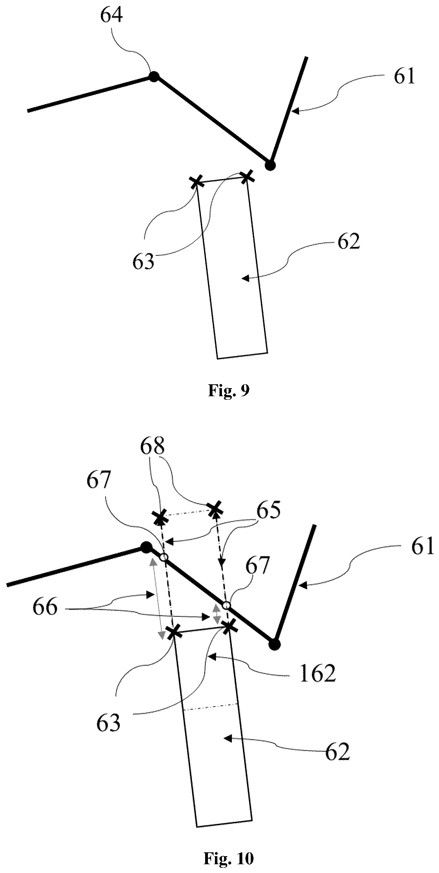

For instance, as illustrated on **Figure 10****,** the first object may correspond to the representation of the surgical tool 62 and the second object may correspond to the representation of the virtual boundary 61. The representation of the surgical tool in its instructed position 162 intersects the representation of the virtual boundary 61 at two intersection points 67. The distances 66 between the control points 63 and the intersection points 67 are then calculated.

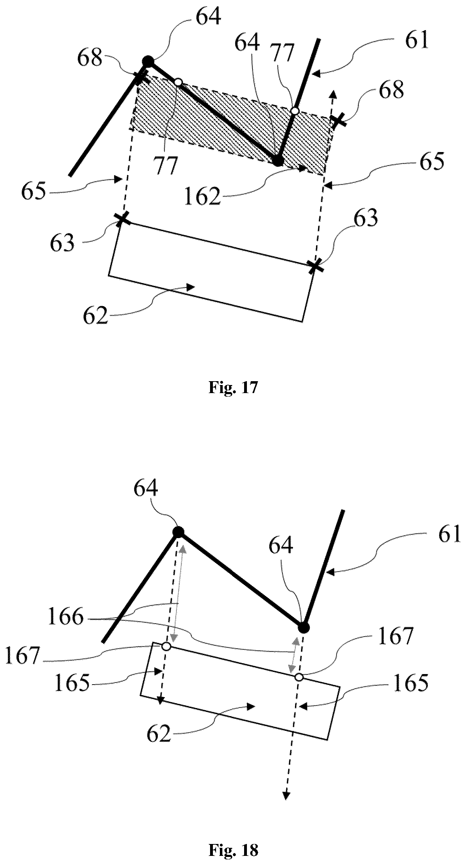

As another example, visible on **Figure 18****,** the first object may correspond to the representation of the virtual boundary 61 and the second object may correspond to the representation of the surgical tool 62. The representation of the virtual boundary 61, when projected onto the representation of the surgical tool 62, using the instructed displacement vectors 165, intersects the representation of the surgical tool 62 at two intersection points 167. The distances 166 between the boundary points 64 and the intersection points 167 are then calculated.

The device 1 may further include a **module 14** configured to compute, for each vertex, the result of a function of said calculated distance. The purpose of such function is to identify the vertex of reference of the first object that will first intersect the second object during the respective movements of the first object and second object.

] The function may be the identity function, in which case the result is simply the calculated distance itself.

Alternatively, the function may be defined as the ratio between the calculated distance and the magnitude of the instructed displacement vector 65, 165 associated with the vertex. In this case, **module 14** may be configured to calculate the magnitude of the instructed displacement vector 65, 165 using the current coordinates of vertices of the first object 21 and the coordinates of the projected first object (e.g., Euclidian distance).

The instructed displacement vector 65, 165 represents the total movement a vertex of the first object is expected to undergo before encountering the second object. The instructed displacement vector 65, 165 defines the intended trajectory of the vertex. The computed ratio quantifies how much of the intended movement is completed before the vertex of the first object interacts with the second object. By comparing these ratios, the system can accurately identify the first vertex of the first object to reach the second object.

This ratio-based approach is particularly advantageous in accounting for rotational movements within the intended trajectory. In a pure translation, all vertices move along a straight path with uniform displacement, making the identity function sufficient for selection. However, when rotation is introduced, vertices follow circular or arc-like paths, causing some points to travel significantly farther than others. As a result, certain vertices may reach the second object earlier in terms of percentage of their instructed displacement, even if their initial direct distance to the second object was greater.

The device 1 may also comprise a **module 15** configured to select a vertex of reference based on the results computed for each vertex in **module 14.**

For instance, the vertex of reference may correspond to the vertex associated with the smallest result of the calculated function. Alternatively, the vertex of reference may correspond to the vertex associated with a result closest to a reference value.

For instance, if the function is the identity function, the vertex of reference may correspond to the vertex associated with the smallest distance among the distances calculated previously.

If the function is the ratio between the distance and the magnitude of the instructed displacement vector 65, 165, the vertex of reference may correspond to the vertex associated with the smallest calculated ratio.

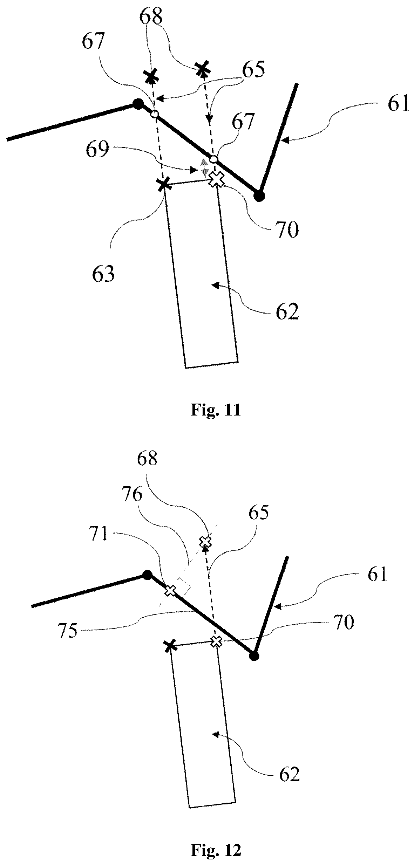

For instance, as illustrated on **Figure 11****,** wherein the first object corresponds to the representation of the surgical tool 62 and the second object corresponds to the representation of the virtual boundary 61, the smallest distance between a control point 63 of the representation of the surgical tool 62 and an intersection point 67 of the representation of the virtual boundary 61 is distance 69. The vertex of reference is therefore a control point of the representation of the surgical tool 62, called control point of reference 70.

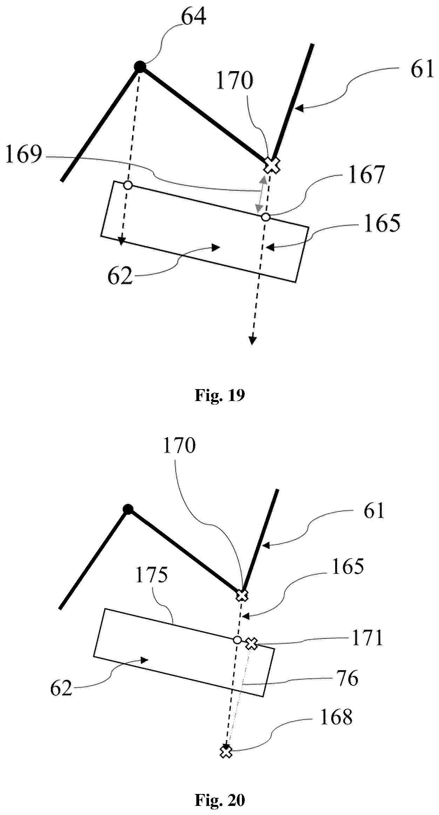

As another example, visible in **Figure 19****,** wherein the first object corresponds to the representation of the virtual boundary 61 and the second object corresponds to the representation of the surgical tool 62, the smallest distance between a boundary point 64 of the representation of the virtual boundary 61 and an intersection point 167 of the representation of the surgical tool 62 is distance 169. The vertex of reference is therefore a boundary point of the representation of the virtual boundary 61, called boundary point of reference 170.

The device 1 may also comprise a **module 16** configured to perform an orthogonal projection of the vertex of reference instructed position (e.g. the vertex of reference position after applying the instructed transformation) on the second object so as to obtain an orthogonal projection point corresponding to the intersection point between the second object and the orthogonal projection axis. The orthogonal projection is performed on an edge of the second object. The edge of the second object selected for the orthogonal projection is generally the first edge crossed by the instructed displacement vector 65 associated with the vertex of reference.

Mathematically, the orthogonal projection involves analyzing the geometric relationship between the control point and the selected edge of the polygon. First, the direction of the edge is determined by calculating the vector between its endpoints. The displacement from the origin of the edge to the control point is then measured. Using the scalar product between these two vectors, a scalar value is calculated that represents how far along the edge's direction the projection of the control point lies. The scalar value is adjusted based on the squared norm (or length) of the edge vector to ensure accurate scaling. Finally, the projection point is determined by adding the appropriately scaled edge vector to the origin of the edge.

As illustrated on **Figure 12****,** wherein the first object corresponds to the representation of the surgical tool 62 and the second object corresponds to the representation of the virtual boundary 61, the instructed displacement vector 65 of the control point 70 crosses a segment 75 of the representation of the virtual boundary 61. Thus, the control point instructed position 68 may be orthogonally projected on the same segment 75. The orthogonal projection axis 76 crosses the segment 75 at an orthogonal projection point 71.

As another example, illustrated on **Figure 20****,** wherein the first object corresponds to the representation of the virtual boundary 61 and the second object corresponds to the representation of the surgical tool 62, the instructed displacement vector 165 of the control point 170 crosses two edges of the representation of the surgical tool 62. The first segment 175 crossed by the instructed displacement vector 165 is selected. Thus, the control point instructed position 168 may be orthogonally projected on the same segment 175. The orthogonal projection axis 76 crosses the segment 175 at an orthogonal projection point 171.

When the instructed displacement vector 65, 165 crosses the second object at a vertex, the edge of the second object selected for the orthogonal projection is the one that forms the smallest angle with the instructed displacement vector 65, 165.

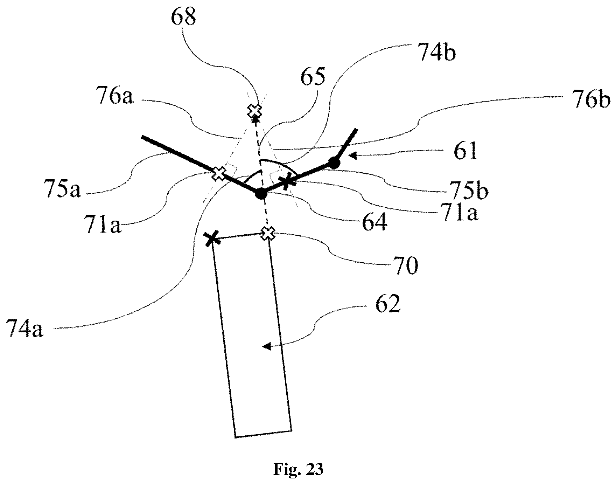

For example, as illustrated on **Figure 23****,** the instructed displacement vector 65 of the control point 70 crosses the representation of the virtual boundary 61 at a boundary point 64, framed by two segments 75a, 75b. The instructed displacement vector 65 forms a first angle 74a with the first segment 75a, while the instructed displacement vector 65 forms a second angle 74b with the second segment 75b. The first angle 74a is smaller than the second angle 74b. Therefore, the first segment 75a is selected as the projection segment. Thus, the control point instructed position 68 may be orthogonally projected on the first segments 75a. The first orthogonal projection axis 76a crosses the first segment 75a at a first orthogonal projection point 71a. The selected orthogonal projection point thus corresponds to the orthogonal projection point 71a.

Device 1 may further comprise a **module 17** configured to update the instructed transformation 20 using the orthogonal projection point and the result of the function of distance obtained for the vertex of reference.

The update of the instructed transformation 20 may be performed in one step or in several iterative steps, depending on the chosen implementation.

For example, if the instructed transformation 20 comprises both a translation component and a rotation component, these can be updated either simultaneously (in one step) or separately (in multiple steps).

In typical 2D transformations, all vertices of the object are affected by the same rotation angle, and thus the same rotation component is applied to all vertices. In other words, there is a single rotation matrix for all vertices. In this case, the rotation component of the instructed transformation 20 may be updated by multiplying each angle of the rotation component by the result of the function of distance. For instance, each angle may be multiplied by the ratio associated with the vertex of reference.

In the context, multiplying the angles of the rotation by the calculated ratio allows for an "interpolation" between the initial and desired final angles. This interpolation helps maintain the intended motion of the polygon while respecting the constraints of the second object. By scaling the rotation component in this manner, the angular change is adjusted proportionally to the portion of the displacement that occurs within the allowed region (i.e. outside of the second object). This ensures that the rotation is not abruptly cut off or altered, providing a smooth transition. However, other approaches can also be employed, such generating a time-dependent trajectory between the initial and desired final angles, for instance, based on a cubic polynomial function.

However, in cases of non-rigid objects, or objects with multiple rotation axes, the vertices may be rotated by different angles, resulting in a different rotation matrix for each vertex. In this case, the rotation component associated with each vertex is updated by multiplying each angle of the rotation component by the result of the function of distance. For instance, each angle may be multiplied by the ratio associated with the vertex of reference.

To update the translation component of the instructed transformation 20, each element of the translation component is updated using the orthogonal projection point coordinates, by computing how much of the instructed translation can actually occur before the second object is reached. Alternatively, the intersection point associated with the vertex of reference could be used directly to update the translation component.

In cases of non-rigid objects, or objects with multiple rotation axes, the vertices may be translated differently, resulting in a different translation matrix for each vertex. In this case, the translation component associated with each vertex is updated independently using the orthogonal projection point.

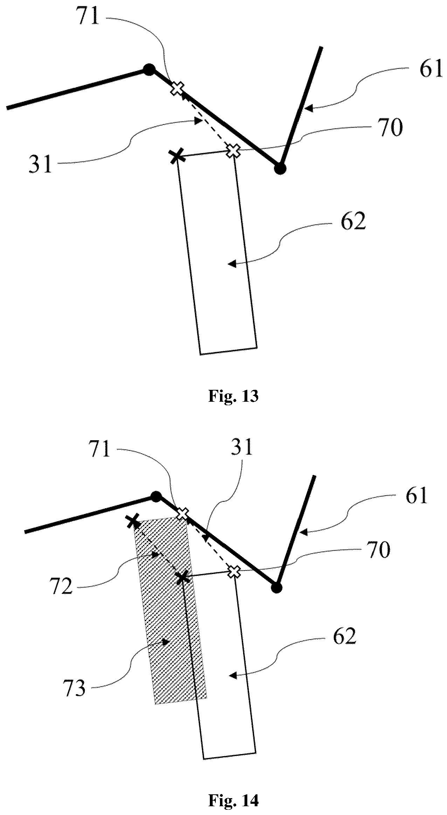

For instance, as illustrated on **Figure 13****,** wherein the first object corresponds to the representation of the surgical tool 62 and the second object corresponds to the representation of the virtual boundary 61, the updated instructed transformation 31 is represented as a vector defined between control point 70 and orthogonal projection point 71.

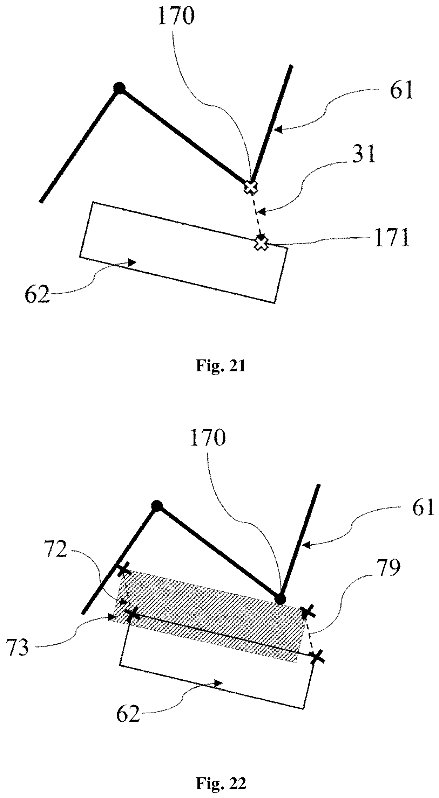

As another example, illustrated on **Figure 21****,** wherein the first object corresponds to the representation of the virtual boundary 61 and the second object corresponds to the representation of the surgical tool 62, the updated instructed transformation 31 is represented as a vector defined between control point 170 and orthogonal projection point 171.

The updated instructed transformation 31 is then outputted by device 1.

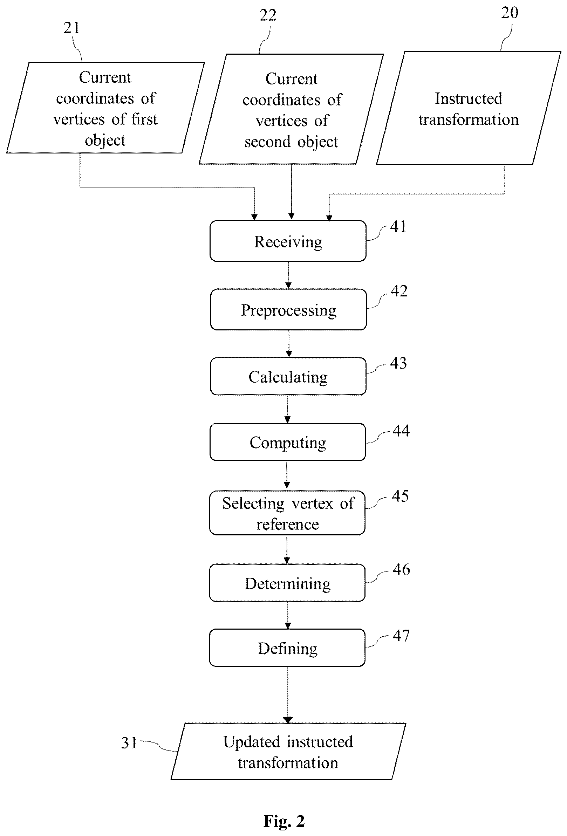

In its automatic actions, the device 1 may for example execute the following process illustrated **on** **Figure 2****:**
- receiving current coordinates of vertices of said first object 21, current coordinates of vertices of said second object 22, and an instructed transformation 20 to be applied to said first object 21 (step 41),
- optionally, preprocessing the current coordinates of vertices of said first object 21, the current coordinates of vertices of said second object 22 and the instructed transformations 20 (step 42),
- for each vertex of the first object, calculating using the current coordinates of the initial position of said vertex 21 and the coordinates of vertices of said second object 22, at least one distance between the initial position of said vertex and an intersection point with at least one edge of the second object along a direction of the instructed displacement vector 65 associated with said vertex (step 43),
- computing a result of a function of said calculated at least one distance (step 44),
- selecting a vertex of reference based on said computed results (step 45),
- determining an orthogonal projection point of an instructed position of said vertex of reference on said at least one edge of the second object comprising the intersection point (step 46)
- updating the instructed transformation 20 using said determined orthogonal projection point and said result of the function of distance obtained for the vertex of reference (step 47), and
- providing said updated instructed transformation 31.

The present invention further relates to a device 2 for determining a corrected transformation 32 for a surgical tool, based on the updated instructed transformation 31 obtained using the device 1. The device 2 will be described in reference to a particular function embodiment as illustrated on **Figure 3****.**

The device 2 may comprise a **module 111** for receiving a current displacement instruction comprising the current coordinates of the control points 24 of the surgical tool (i.e., the coordinates before a new displacement of the surgical tool, in the initial position of the surgical tool), the current coordinates of the boundary points 25 and, an initial transformation 27 to be applied to the current coordinates of control points 24 of the surgical tool to displace the representation of the surgical tool from its initial position to an instructed position.

According to the invention, control points are specific reference points used to mathematically model and monitor a surgical tool's position and orientation in a virtual environment. These control points are typically predefined by the tool's manufacturer and correspond to distinct locations on the surgical tool, such as its tip, edges, or other strategic parts of its geometry. The control points may be used to create a polygonal or geometric representation of the surgical tool, allowing for accurate tracking and manipulation during the procedure.

In the same manner, boundary points are specific reference points used to mathematically model and monitor a virtual boundary's position and orientation in a virtual environment. The virtual boundary may be defined by the surgeon during the operation or preoperatively. The virtual boundary may remain unchanged during the whole operation or evolve during the surgical operation.

The current coordinates of the boundary points 25 and the current coordinates of the control points 24 of the surgical tool may be 2D coordinates. They may be provided in various formats, depending on the application and data structure. For instance, the coordinates may be cartesian coordinates or polar coordinates, represented in lists, arrays, or matrices or in text-based formats like CSV, JSON, or XML; or in geometric formats like SVG or CAD files. As a particular example, current coordinates of the boundary points 25 and the current coordinates of the control points 24 may be expressed relative to the origin of a coordinate system, potentially using transformations that describe their positions and orientations in that system.

The initial transformation 27 may be expressed as a transformation matrix configured to be applied to the control points of the surgical tool to compute the instructed position of the surgical tool. The initial transformation 27 may include a translation component. The initial transformation 27 may further include a rotation component.

In some embodiments, the initial transformation 27 may be represented as a single transformation matrix that integrates both translation and rotation components. In other embodiments, the initial transformation 27 may be decomposed into multiple matrices, each corresponding to a distinct transformation operation. For instance, a first matrix may define a translation transformation, a second matrix may define a rotation transformation. These matrices may be applied sequentially, either in a predetermined order or based on specific transformation parameters, to compute the instructed position of the surgical tool.

All these input data may be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

**Module 112** may be optionally configured to perform a preprocessing of the input data. For example, preprocessing may include filtering out redundant or irrelevant coordinates or normalizing the input data to a common reference frame.

The device 2 may also include a **module 113** configured to project the representation of the surgical tool (in its initial position) and the representation of the virtual boundary in a common virtual referential using the coordinates received as input. As illustrated on **Figure** 9, at this step, the representation of the surgical tool 62 may be a rectangle (e.g. when the surgical tool is a bone saw or a drill) comprising two control points 63 positioned at the distal end of the surgical tool. The representation of the virtual boundary (only partially represented on **Figure** 9) comprises boundary points 64 connected by edges.

**Module 113** may further be configured to displace the representation of the surgical tool into the instructed position by applying the initial transformation 27 to the control points of the representation of the surgical tool.

To facilitate understanding, the initial transformation 27 induced displacement is represented on the figures as instructed displacement vectors 65 applied to the control points.

For instance, as illustrated on **Figure 10****,** wherein the surgical tool is a rigid, non-deformable tool, an identical displacement vector 65 is applied to each control point 63 of the representation of the surgical tool 62. The control points 63 are displaced from their initial position to an instructed position 68. A displaced representation of the surgical tool 162 (i.e., surgical tool in its instructed position 162) is thus obtained.

The device 2 may further include a **module 114** configured to determine whether the representation of the surgical tool in its instructed position crosses the representation of the virtual boundary. A point-in-polygon algorithm may for instance be implemented to determine if there is an intersection.

If the representation of the surgical tool in its instructed position does not intersect the virtual boundary, no issue is detected. The device 2 may therefore include a **module 115** configured to define the current displacement instruction received as input as the corrected instruction. This corrected instruction may then be applied (e.g., to the surgical tool, for instance via the robotic arm).

However, if the representation of the surgical tool in its instructed position intersects the virtual boundary, a corrected instruction shall be determined.

For instance, in **Figure 11****,** the representation of the surgical tool 62 in its instructed position crosses the representation of the virtual boundary 61 in two points 67. Moreover, the control points 68 are localized inside of the representation of the virtual boundary 61.

The device 2 may comprise a **module 116** configured to provide as an input to the device 1, the data received as input by device 2 (e.g., the current coordinates of control points 24, the current coordinates of boundary points 25 and the initial transformation 27) and to receive the updated instructed transformation 31 outputted by the device 1.

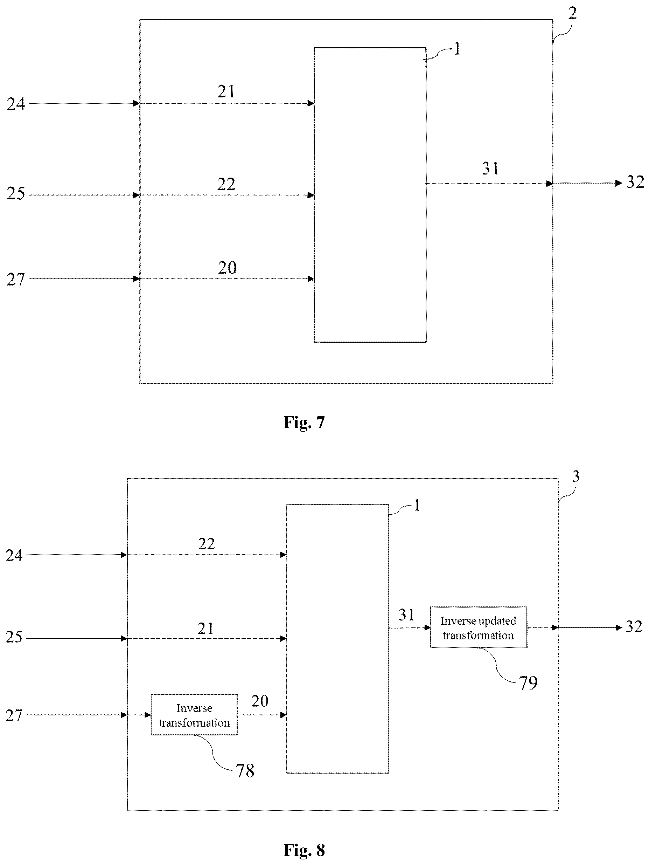

As illustrated on **Figure 7****,** device 2 uses device 1 in such way that the first object corresponds to the representation of the surgical tool and the second object corresponds to the representation of the virtual boundary. Therefore, the current coordinates of the control points 24 of the surgical tool are provided as input to device 1 and correspond to the current coordinates of vertices of the first object 21. The current coordinates of the boundary points 25 are provided as input to the device 1 and correspond to the current coordinates of vertices of the second object 22 and the initial transformation 27 to be applied to the control points is provided as input to the device 1 and corresponds to the instructed transformation 20.

As illustrated on **Figure 14****,** the obtained corrected transformation 32, corresponding to the instructed transformation 20 outputted by device 1, may be used on the representation of the surgical tool 62, to obtain a corrected position of the representation of the surgical tool, wherein it does not cross the representation of the virtual boundary 61.

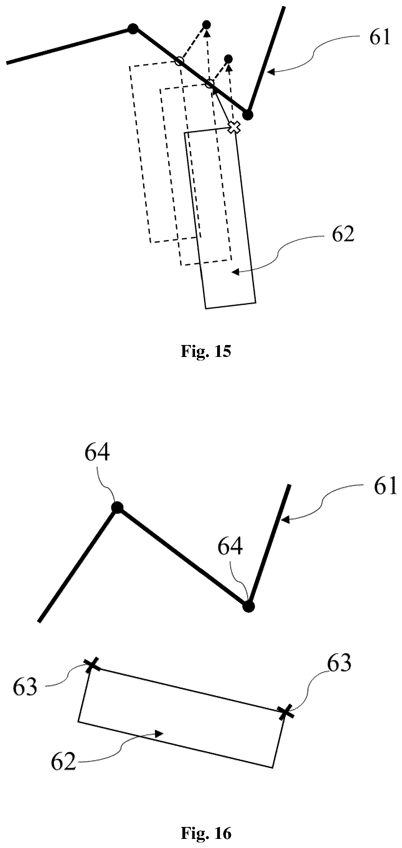

As illustrated on **Figure 15****,** the steps executed by **module 111 to module 116** may be repeated multiple times in such way that the representation of the surgical tool 62 follows the representation of the virtual boundary 61 without crossing it.

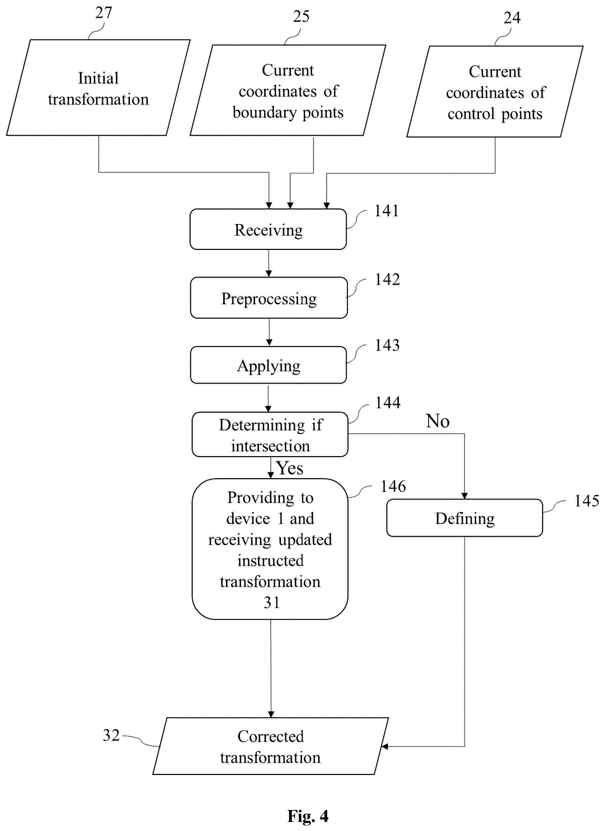

In its automatic actions, the device 2 may for example execute the following process illustrated on **Figure 4****:**
- receiving a current displacement instruction comprising the current coordinates of control points 24, the current coordinates of boundary points 25 and the initial transformation 27 to be applied to the control points (step 141),
- optionally preprocessing the current coordinates of control points 24, the current coordinates of boundary points 25 and the initial transformation 27 (step 142),
- applying the initial transformation 27 to said representation of the surgical tool so as to obtain a displaced representation of said surgical tool (step 143)
- determining if said displaced representation of said surgical tool intersects said representation of said virtual boundary (step 144)
- if the displaced representation of said surgical tool does not intersect the representation of the virtual boundary, define the corrected transformation 32 as the initial transformation 27 (step 145),
- if the displaced representation of said surgical tool does intersect the representation of the virtual boundary, providing as input to the device 1, the current coordinates of control points 24, the current coordinates of boundary points 25 and the initial transformation 27, wherein the first object corresponds to the representation of the surgical tool and wherein the second object corresponds to the representation of the virtual boundary, so as to obtain the updated instructed transformation 31 and obtaining as an output an updated instructed transformation 31 (step 146),
- outputting said updated instructed transformation 31 as the corrected transformation 32.

The present invention further relates to a device 3 for determining a corrected transformation 32 for a surgical tool, based on the updated instructed transformation 31 obtained using the device 1. The device 3 will be described in reference to a particular function embodiment as illustrated on **Figure 5****.**

The device 3 may comprise a **module 121** for receiving a current displacement instruction comprising the current coordinates of the control points 24 of the surgical tool (i.e., the coordinates before a new displacement of the surgical tool, in the initial position of the surgical tool), the current coordinates of the boundary points 25 and an initial transformation 27 to be applied to the control points of the surgical tool to displace the representation of the surgical tool from its initial position to an instructed position.

The current coordinates of the boundary points 25 and the current coordinates of the control points 24 of the surgical tool may be 2D coordinates. They may be provided in various formats, depending on the application and data structure. For instance, the coordinates may be cartesian coordinates or polar coordinates, represented in lists, arrays, or matrices or in text-based formats like CSV, JSON, or XML; or in geometric formats like SVG or CAD files. As a particular example, current coordinates of the boundary points 25 and the current coordinates of the control points 24 may be expressed relative to the origin of a coordinate system, potentially using transformations that describe their positions and orientations in that system.

The initial transformation 27 may be expressed as a transformation matrix configured to be applied to the control points of the surgical tool to compute the instructed position of the surgical tool. The initial transformation 27 may include a translation component. The initial transformation 27 may further include a rotation component.

In some embodiments, the initial transformation 27 may be represented as a single transformation matrix that integrates both translation and rotation components. In other embodiments, the initial transformation 27 may be decomposed into multiple matrices, each corresponding to a distinct transformation operation. For instance, a first matrix may define a translation transformation, a second matrix may define a rotation transformation. These matrices may be applied sequentially, either in a predetermined order or based on specific transformation parameters, to compute the instructed position.

All these input data may be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

**Module 122** may be optionally configured to perform a preprocessing of the input data. For example, preprocessing may include filtering out redundant or irrelevant coordinates or normalizing the input data to a common reference frame.

The device 3 may also include a **module 123** configured to project the representation of the surgical tool (in its initial position) and the representation of the virtual boundary in a common virtual referential using the coordinates received as input. As illustrated on **Figure 16****,** at this step, the representation of the surgical tool 62 may be a rectangle (e.g. when the surgical tool is a bone saw or a drill) comprising two control points 63. The representation of the virtual boundary (only partially represented on **Figure 16****)** comprises boundary points 64 connected by edges.

**Module 123** may further be configured to displace the representation of the surgical tool into the instructed position by applying the initial transformation 27 to the control points of the representation of the surgical tool.

To facilitate understanding, the initial transformation 27 induced displacement is represented on the figures as instructed displacement vectors 65 applied to the control points.

For instance, as illustrated on **Figure 17****,** wherein the surgical tool is a rigid, non-deformable tool, an identical displacement vector 65 is applied to each control point 63 of the representation of the surgical tool 62. The control points 63 are displaced from their initial position to an instructed position 68.

The device 3 may further include a **module 124** configured to determine whether the representation of the surgical tool in its instructed position crosses the representation of the virtual boundary. A point-in-polygon algorithm may for instance be implemented to determine if there is an intersection.

If the representation of the surgical tool in its instructed position does not intersect the virtual boundary, no issue is detected. The device 3 may therefore include a **module 125** configured to define the current displacement instruction received as input as the corrected instruction. This corrected instruction may then be applied (e.g., to the surgical tool, for instance via the robotic arm).

However, if the representation of the surgical tool in its instructed position intersects the virtual boundary, a corrected instruction shall be determined.

For instance, in **Figure 17****,** the representation of the surgical tool 62 in its instructed position crosses the representation of the virtual boundary 61 in two points 77. These points 77 are localized on edges of the representation of the surgical tool 62. However, none of the control points 68 are localized inside of the representation of the virtual boundary 61.

The device 3 may comprise a **module 126** for calculating the inverse transformation 78 of the initial transformation 27.

Notably, in the case of an isometric transformation, the inverse transformation T⁻¹ of a transformation T is obtained by applying the transpose to a rotational component of the transformation and the negation to a translation component.

The device 3 may comprise a **module 127** for providing as an input to the device 1, the current coordinates of control points 24, the current coordinates of boundary points 25 and the inverse transformation calculated by module 126. Module 126 is then configured to receive the updated instructed transformation 31 outputted by the device 1.

As illustrated on **Figure 8****,** device 3 uses device 1 in such way that the first object corresponds to the representation of the virtual boundary and the second object corresponds to the representation of the surgical tool. Therefore, the current coordinates of the control points 24 of the surgical tool are provided as input to device 1 and correspond to the current coordinates of vertices of the second object 22. The current coordinates of the boundary points 25 are provided as input to the device 1 and correspond to the current coordinates of vertices of the first object 21 and the inverse transformation 78 to be applied to the control points is provided as input to the device 1 and corresponds to the instructed transformation 20.

The device 3 may further comprise a **module 128** for calculating an inverse updated transformation 79 using the updated instructed transformation 31 outputted by device 1.

As illustrated on **Figure 22****,** the obtained corrected transformation 32, corresponding to the inverse updated transformation 79, may be used on the representation of the surgical tool 62, to obtain a corrected position of the representation of the surgical tool, wherein it does not cross the representation of the virtual boundary 61.

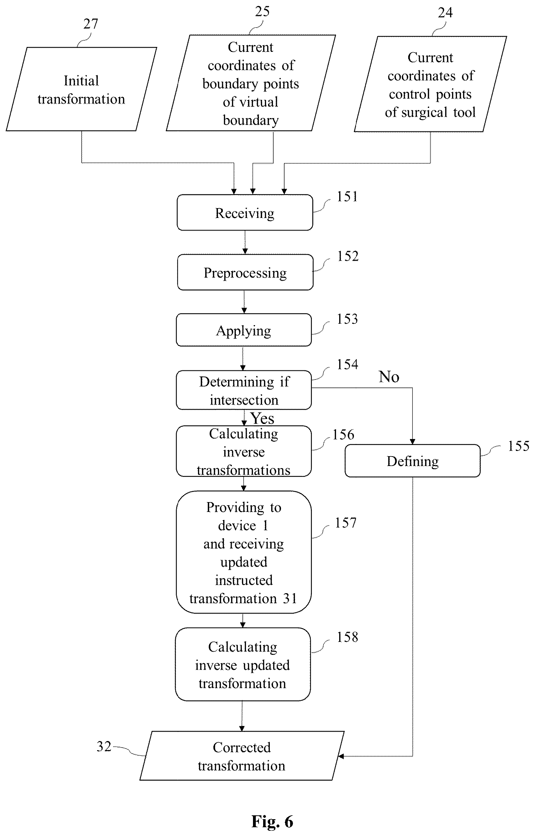

In its automatic actions, the device 3 may for example execute the following process illustrated on **Figure 6****:**
- receiving a current displacement instruction comprising the current coordinates of control points 24 of the surgical tool, the current coordinates of boundary points 25 and the initial transformation 27 to be applied to the control points (step 151),
- optionally preprocessing the current coordinates of control points 24 of the surgical tool, the current coordinates of boundary points 25, and the initial transformation 27 (step 152),
- applying the initial transformation 27 so as to obtain a displaced representation of said surgical tool (step 153),
- determining if said displaced representation of said surgical tool intersects said representation of said virtual boundary (step 154)
- if the displaced representation of said surgical tool does not intersect the representation of the virtual boundary, define the corrected transformation 32 as the initial transformation 27 (step 155),
- if the displaced representation of said surgical tool does intersect the representation of the virtual boundary, calculating an inverse transformation 78 of the initial transformation 27 (step 156),
   o providing as input to the device 1, the current coordinates of control points 24 of the surgical tool, the current coordinates of boundary points 25 and the inverse transformation, wherein the first object corresponds to the representation of the surgical tool and wherein the second object corresponds to the representation of the virtual boundary, so as to obtain the updated instructed transformation 31 (step 157),
- calculating an inverse updated transformation 79 using the updated instructed transformation (step 158),
- outputting said inverse updated transformation 79 as the corrected transformation 32.

The device 2 and the device 3 may be articulated in several ways as they verify different scenarios (i.e., first scenario: during the movements of the representation of the surgical tool, one of its vertices intersects the representation of the virtual boundary, and second scenario: during the movements of the polygonal representation of the surgical tool, one of its edges intersects the virtual boundary but none of its vertices crosses the virtual boundary). The device 1 and the device 2 may be run simultaneously so as to obtain two corrected transformations 32. In this case, one corrected transformation 32 may be selected depending on at least one specific criterion. The criteria may be selected depending on the accuracy of the correction (e.g., smoothness of the resulting movement), the minimization of the computational effort, the surgical context, etc.

Alternatively, devices 1 and 2 may be run sequentially, such that the corrected transformation 32 outputted by one of the devices 2 or 3 is reinjected into the other device 3 or 2 as the initial transformation 27.

The above-described devices 1, 2, 3 are implemented in a 2D space because the 3D representation of the virtual boundary and the 3D representation of the surgical tool are projected onto a 2D surface (e.g., defined parallel to a surgical plan, for example parallel to the floor of the operating room). To reconstruct the 3D representations of the virtual boundary and the 3D representations of the surgical tool, a mapping process is employed, where the 2D polygons are converted into 3D surfaces, for example composed of triangles.

After determining the corrected transformation 32, the devices 2, 3 may be further configured to determine the "real position" of the surgical tool (e.g., its actual spatial location and orientation in the physical operating environment) relative to the position of the control points as received by the devices 2, 3. The real position is typically derived by applying a transformation matrix to the control points, ensuring alignment with the surgical plan and/or patient anatomy.

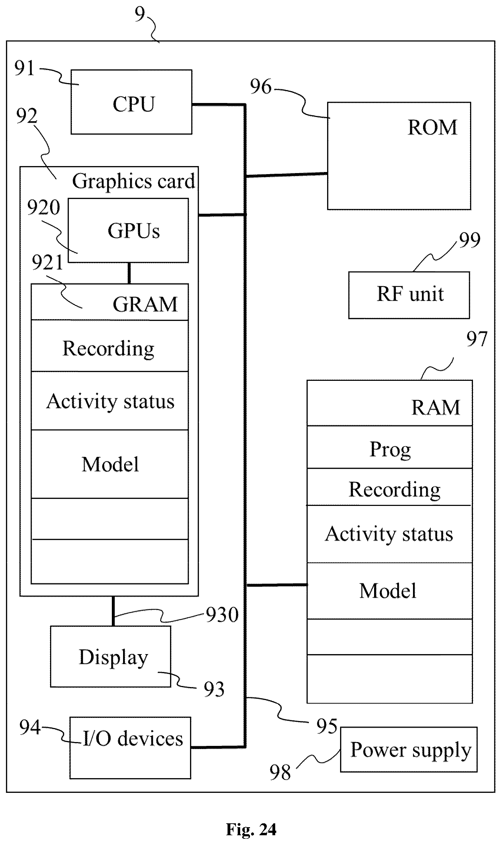

A particular apparatus 9, visible on **Figure 24** is embodying the devices 1, 2 and/or 3 described above. It corresponds for example to a workstation, a laptop, a tablet, or a smartphone, a PC connected to a robotic arm controller.

That apparatus 9 may comprise the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 may also comprise a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed such as image data 971 or the patient-specific 3D model 972). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program 970 contained in the RAM 97.

As will be understood by a skilled person, the presence of the graphics card 92 is not mandatory and can be replaced with entire CPU processing and/or simpler visualization implementations.

In variant modes, the apparatus 9 may include only the functionalities of the device 1. In addition, the device 1 may be implemented differently than a standalone software, and an apparatus or set of apparatus comprising only parts of the apparatus 9 may be exploited through an API call or via a cloud interface.

Notably, the above-described devices and methods may be integrated within a surgical system (not represented in the figures) comprising at least one of a robotic arm, an imaging system and a localization system.

The imaging system may be an X-ray imaging system, such as a C-shaped arm, that performs computed tomography (CT) by capturing images from different projection angles. These images are then used to reconstruct a 3D volume of the anatomical structure of interest.

The localization system may be capable of tracking the position and orientation of various components in real time. This localization system is designed to track trackers attached to the anatomical structure of interest, surgical tools, and the robotic arm. It may use technologies such as optical, electromagnetic, or ultrasound-based tracking, enabling accurate real-time localization of both the surgical tool and the anatomical structure. For example, optical trackers with reflective markers may be used to localize the position and orientation of trackers mounted rigidly on the patient, robotic arm, or surgical tools.

The robotic arm may comprise a movable cart. The cart is configured to remain fixed relative to the operating table while the robotic arm moves to reach a surgical target. The cart can be either manually or motorized for movement, with features such as lockable wheels to stabilize the system during the procedure. The robotic arm may offer multiple degrees of freedom in translation and rotation (e.g., equipped with six or more motorized axes such as KUKA or KINOVA models).

The robotic arm may comprise an end effector designed to hold medical devices such as surgical tools or guides. This end effector is mechanically coupled to the distal end of the robotic arm and can be moved with high accuracy to assist the surgeon in positioning and guiding the surgical tool with the required orientation.

The robotic arm may be equipped with a computing unit that manages the robotic arm movements. This control unit includes at least one processor, a data storage device, and a communication device, and may be embedded in the movable cart. The control unit may also be responsible for controlling the movement of the cart. The control unit may be communicatively coupled with device 2 and/or device 3 to receive a corrected transformation 32 and implement the corrected transformation 32 to control movements of the robotic arm so as to maintain the surgical tool within the virtual boundary.

## Claims

1. A device (1) for providing an updated instructed transformation (31) for preventing a first object from crossing a second object, wherein said updated instructed transformation (31) is configured to be used to ensure controlled interaction between a surgical tool and a virtual boundary that delineates a target surgical area, such that the virtual boundary is respected and/or integrated into a trajectory of the surgical tool, said device (1) comprising:
- at least one input configured to receive:
o current coordinates of vertices of said first object (21), said vertices being in an initial position, at least two of said vertices being connected by an edge to form said first object;
o current coordinates of vertices of said second object (22), at least two of said vertices being connected by an edge to form said second object;
o an instructed transformation (20) to be applied to said vertices of said first object (21) to displace the vertices from the initial position to an instructed position, said instructed transformation (20) defining for each vertex of said first object, an associated instructed displacement vector (65) between said initial position of said vertex and an instructed position of said vertex;
- at least one processor configured to:
o for each vertex of the first object:
▪ calculate (13), using the current coordinates of the initial position of said vertex (21) and the coordinates of vertices of said second object (22), at least one distance between the initial position of said vertex and an intersection point with at least one edge of the second object along a direction of the instructed displacement vector (65) associated with said vertex,
▪ compute (14) a result of a function of said calculated at least one distance,
o select (15) a vertex of reference based on said computed results,
o determine (16) an orthogonal projection point of an instructed position of said vertex of reference on said at least one edge of the second object comprising the intersection point,
o update (17) the instructed transformation (20) using said determined orthogonal projection point and said result of the function of distance obtained for the vertex of reference,
- at least one output configured to provide said updated instructed transformation (31).

2. The device according to claim **1,** wherein when the instructed displacement vector (65) associated with the vertex of reference intersects a vertex of the second object, said vertex of the second object being an intersection between two edges of the second object, including a first edge and a second edge, the instructed displacement vector (65) forming a first angle with the first edge and the instructed displacement vector (65) forming a second angle with the second edge, said at least one processor is further configured to:
- select, between the first edge and the second edge, an edge forming a smallest angle with the instructed displacement vector (65), between the first angle and the second angle,
- calculate the at least one distance using the selected edge.

3. A device (2) for determining a corrected transformation (32) for a surgical tool, based on an updated instructed transformation (31) obtained using the device from claim **1 or 2,** said device (2) comprising:
- at least one input configured to receive a current displacement instruction comprising:
o current coordinates of control points (24) corresponding to an initial position of said surgical tool, wherein said control points are associated to predefined locations on the surgical tool and at least two control points are connected by an edge to form a representation of the surgical tool;
o current coordinates of boundary points (25), said boundary points delimitating a virtual boundary, at least two boundary points being connected by a boundary segment to form a representation of the virtual boundary;
o an initial transformation (27) to be applied to the current coordinates of control points (24),
- at least one processor configured to:
o apply the initial transformation (27) to said control points, so as to obtain a displaced representation of said surgical tool,
o determine if the displaced representation of the surgical tool intersects the representation of the virtual boundary:
▪ if said displaced representation of the surgical tool does not intersect said representation of the virtual boundary, define said corrected transformation (32) as said initial transformation (27),
▪ if said displaced representation of the surgical tool does intersect the representation of the virtual boundary:
• provide as an input to a device according to claim **1 or 2** said current coordinates of control points (24) as current coordinates of vertices of said first object (21), said current coordinates of boundary points (25) as current coordinates of vertices of said second object (22), and said initial transformation (27) as instructed transformation (20), so that the first object corresponds to the representation of the surgical tool and the second object corresponds to the representation of the virtual boundary;
• obtain as an output an updated instructed transformation (31),
- at least one output configured to provide said updated instructed transformation as the corrected transformation (32).

4. A device (3) for determining a corrected transformation (32) for a surgical tool, based on an updated instructed transformation (31) obtained using the device from claim **1 or 2,** said device (3) comprising:
- at least one input configured to receive a current displacement instruction comprising:
o current coordinates of control points (24) corresponding to an initial position of said surgical tool, wherein said control points are associated to predefined locations on the surgical tool and at least two control points are connected by an edge to form a representation of the surgical tool;
o current coordinates of boundary points (25), said boundary points delimitating a virtual boundary, at least two boundary points being connected by a boundary segment to form a representation of the virtual boundary;
o an initial transformation (27) to be applied to the current coordinates of control points (24),
- at least one processor configured to:
o apply the initial transformation (27) to said control points so as to obtain a displaced representation of said surgical tool,
o determine if the displaced representation of the surgical tool intersects the representation of the virtual boundary:
▪ if said displaced representation of the surgical tool does not intersect said representation of the virtual boundary, define said corrected transformation (32) as said initial transformation (27),
▪ if said projected representation of the surgical tool does intersect the representation of the virtual boundary:
• calculate an inverse transformation (78) of the initial transformation (27),
• provide as an input to a device according to claim **1 or 2** said current coordinates of boundary points (25) as current coordinates of vertices of said first object (21), said current coordinates of control points (24) as current coordinates of vertices of said second object (22), and said inverse transformations (78) as instructed transformation (20), so that the first object corresponds to the representation of the virtual boundary and the second object corresponds to the representation of the surgical tool,
• obtain as an output an updated instructed transformation (31),
• calculate an inverse updated transformation (79) using the updated instructed transformation (31),
- at least one output configured to provide said inverse updated transformation (79) as said corrected transformation (32).

5. Ensemble comprising the device (2) according to claim 3 and the device (3) according to claim **4,** wherein said ensemble comprises:
- at least one processor communicatively coupled to the device (2) and the device (3), said at least one processor being configured to send an execution request to either one of the device (2) and the device (3) so as to obtain as output a first corrected transformation, said at least one processor being further configured to provide said first corrected transformation as the initial transformation (27) of the other of the two devices, between device (2) and device (3), so as to obtain a second corrected transformation,
- at least one output configured to provide said corrected transformation.

6. Ensemble comprising the device (2) according to claim 3 and the device (3) according to claim **4,** wherein said ensemble comprises:
- at least one processor communicatively coupled to the device (2) and the device (3), said at least one processor being configured to send an execution request to the device (2) and the device (3) so as to obtain a third corrected transformation as output of the device (2) and a fourth corrected transformation as output of the device (3), said at least one processor being further configured to select a corrected transformation between the third corrected transformation and the fourth corrected transformation with respect to a criterion, wherein said criterion corresponds to a minimization of the update to the initial transformation (27), and
- at least one output configured to provide the selected corrected transformation.

7. The ensemble according to claim **5** or **6,** wherein said at least one input is configured to periodically receive a new current displacement instruction, said at least one processor being configured to calculate a corrected transformation each time a new current displacement instruction is received.

8. The ensemble according to claim **5** or **6,** wherein if the displaced representation of said control points is determined to intersect the representation of the virtual boundary, the at least one processor is configured to implement a safety measure to prevent further deviation and restore compliance with the representation of the virtual boundary.

9. A surgical system comprising:
- a robotic arm configured to control a trajectory of at least one surgical tool, said robotic arm comprising at least one processor configured to receive a movement instruction initiated by a user manipulating the robotic arm, said at least one processor being configured to determine said initial transformation (27) based on said received movement instruction,
- the ensemble according to any of claims 5 to 8, wherein said ensemble is configured to receive as input the initial transformation (27) determined by said at least one processor and to output a corrected transformation, said at least one processor being configured to implement the corrected transformation to control movements of the robotic arm so as to maintain the surgical tool within the virtual boundary.

10. A method for providing an updated instructed transformation (31) for preventing a first object from crossing a second object, wherein said updated instructed transformation (31) is configured to be used to ensure controlled interaction between a surgical tool and a virtual boundary that delineates a target surgical area, such that the virtual boundary is respected and/or integrated into a trajectory of the surgical tool, said method comprising:
- receiving:
o current coordinates of vertices of said first object (21), said vertices being in an initial position, at least two of said vertices being connected by an edge to form said first object;
o current coordinates of vertices of said second object (22), at least two of said vertices being connected by an edge to form said second object;
o an instructed transformation (20) to be applied to said vertices of said first object (21) to displace the vertices from the initial position to an instructed position, said instructed transformation (20) defining for each vertex of said first object, an associated instructed displacement vector (65) between said initial position of said vertex and an instructed position of said vertex;
- for each vertex of the first object:
o calculating, using the current coordinates of the initial position of said vertex (21) and the coordinates of vertices of said second object (22), at least one distance between the initial position of said vertex and an intersection point with at least one edge of the second object along a direction (23) of the instructed displacement vector (65) associated with said vertex,
o computing a result of a function of said calculated at least one distance,
- selecting a vertex of reference based on said computed results,
- determining an orthogonal projection point of an instructed position of said vertex of reference on said at least one edge of the second object comprising the intersection point,
- updating the instructed transformation (20) using said determined orthogonal projection point and said result of the function of distance obtained for the vertex of reference,
- providing said updated instructed transformation (31).

11. A method for determining a corrected transformation (32) for a surgical tool, based on an updated instructed transformation (31) obtained using the device from claim **1** or **2,** said method comprising:
- receiving a current displacement instruction comprising:
o current coordinates of control points (24) corresponding to an initial position of said surgical tool, wherein said control points are associated to predefined locations on the surgical tool and at least two control points are connected by an edge to form a representation of the surgical tool;
o current coordinates of boundary points (25), said boundary points delimitating a virtual boundary, at least two boundary points being connected by a boundary segment to form a representation of the virtual boundary;
o an initial transformation (27) to be applied to the current coordinates of control points (24),
- applying the initial transformation (27) to said control points, so as to obtain a displaced representation of said surgical tool,
- determining if the displaced representation of the surgical tool intersects the representation of the virtual boundary:
o if said displaced representation of the surgical tool does not intersect said representation of the virtual boundary, defining said corrected transformation (32) as said initial transformation (27),
o if said displaced representation of the surgical tool does intersect the representation of the virtual boundary:
▪ providing as an input to a device according to claim **1** or **2** said current coordinates of control points (24) as current coordinates of vertices of said first object (21), said current coordinates of boundary points (25) as current coordinates of vertices of said second object (22), and said initial transformation (27) as instructed transformation (20), so that the first object corresponds to the representation of the surgical tool and the second object corresponds to the representation of the virtual boundary;
▪ obtaining as an output an updated instructed transformation (31),
- providing said updated instructed transformation (31) as the corrected transformation (32).

12. A method for determining a corrected transformation (32) for a surgical tool, based on an updated instructed transformation (31) obtained using the device from claim **1** or **2,** said method comprising:
- receiving a current displacement instruction comprising:
o current coordinates of control points (24) corresponding to an initial position of said surgical tool, wherein said control points are associated to predefined locations on the surgical tool and at least two control points are connected by an edge to form a representation of the surgical tool;
o current coordinates of boundary points (25), said boundary points delimitating a virtual boundary, at least two boundary points being connected by a boundary segment to form a representation of the virtual boundary;
o an initial transformation (27) to be applied to the current coordinates of control points (24),
- applying the initial transformation (27) to said control points so as to obtain a displaced representation of said surgical tool,
- determining if the displaced representation of the surgical tool intersects the representation of the virtual boundary:
o if said displaced representation of the surgical tool does not intersect said representation of the virtual boundary, defining said corrected transformation (32) as said initial transformation (27),
o if said projected representation of the surgical tool does intersect the representation of the virtual boundary:
▪ calculating an inverse transformation (78) of the initial transformation (27),
▪ providing as an input to a device according to claim **1 or 2** said current coordinates of boundary points (25) as current coordinates of vertices of said first object (21), said current coordinates of control points (24) as current coordinates of vertices of said second object (22), and said inverse transformations (78) as instructed transformation (20), so that the first object corresponds to the representation of the virtual boundary and the second object corresponds to the representation of the surgical tool,
▪ obtaining as an output an updated instructed transformation (31),
▪ calculating an inverse updated transformation (79) using the updated instructed transformation (31),
- providing said inverse updated transformation (79) as said corrected transformation (32).

13. A computer program product comprising instructions which, when the program is executed by a computer, cause the computer to carry out at least one of the methods of claims **10** to **12.**

14. A computer-readable storage medium comprising instructions which, when executed by a computer, cause the computer to carry out at least one of the methods of claims **10** to **12.**
